# EUROPEAN PATENT SPECIFICATION

(11) **EP 2 983 567 B1**
(45) Date of publication and mention of the grant of the patent: **07.06.2017**
(21) Application number: 13779160.4
(22) Date of filing: 04.10.2013
(51) Int. Cl.: A47G 9/00, A61H 15/00

(54) **BLANKET WITH CHANNELS FILLED WITH SUBSTANTIALLY SPHERICAL OBJECTS FOR THERAPEUTIC TREATMENT**
DECKE MIT KANÄLEN MIT FÜLLUNG AUS WEITGEHEND KUGELFÖRMIGEN GEGENSTÄNDEN ZUR THERAPEUTISCHEN BEHANDLUNG
COUVERTURE PRÉSENTANT DES CANAUX REMPLIS D'OBJETS SENSIBLEMENT SPHÉRIQUES POUR UN TRAITEMENT THÉRAPEUTIQUE PERSONNALISÉ

(30) Priority: 09.04.2013 DK 201300055
(43) Date of publication of application: 17.02.2016
(73) Proprietor: Protac A/S, 8660 Skanderborg (DK)
(72) Inventor: CHRISTIANSEN, Pia Riishøj, 8000 Aarhus C (DK); PEDERSEN, Birthe Søndergaard, 8680 Ry (DK)
(74) Representative: Zacco Denmark A/S
(86) International application number: PCT/EP2013/070690
(87) International publication number: WO 2014/166556

(56) References cited:
- WO-A1-2009/123557
- GB-A- 2 304 571
- GB-A- 2 353 711
- US-A- 2 628 046
- US-A- 5 105 490

## Description

### Technical field of the invention

The present invention relates to therapeutic blankets. More specifically, the invention relates to a therapeutic blanket with channels filled with substantially spherical objects.

### Background of the invention

It is known that persons who suffer from angst, unrest, or sensory disorders have difficulty in finding rest, sleeping, or relaxing. A reason for this may be that they have difficulty in feeling the boundaries of their own bodies, and they can need a physical object to focus on, preferably something that presses against the body and that they can bend and wrap around themselves. Especially for hyperactive, restless children or children with an ADHD problem or Asperger's syndrome, a physical object makes a great difference when calm, sleep, or insufficient relaxation is involved, but also children and adults with a general complaint of sleep problems, unrest, and angst can benefit from a simple and practical solution to relieve the complaint.

By pressure applied to the body, the user receives a feeling of safety, and it is believed that a number of bodily substances are released that have a calming effect, including oxytocin. If it is possible to follow the form of the body and to vary the pressure between certain points, this contributes to tactile stimulation and can increase the positive result for the user.

It is already known to use filled blankets for this purpose. Some variants show a blanket that contains plastic pellets or balls. These materials all have the common feature that they are based on essentially point-like objects. In order to keep the fillings from moving to the lowest point of the blanket during use, patches, preferably with a square shape, are often used. However, such a patch filling result in a relatively thick blanket having good insulating properties. E.g. in summertime, this is not always convenient. Furthermore, the thickness of the blanket makes it difficult to fold up or handle, and cleaning and washing can be made difficult or completely impossible.

EP 0,729,340 discloses a blanket for therapeutic use having essentially square patches filled with therapy balls. The balls used have a diameter of about 50 mm, and they exert a more point-shaped pressure against the body, which is believed to increase the release of favourable bodily substances and thereby have a positive effect on the user.

WO2009123557 discloses a blanket for therapeutic treatment, the blanket having a first face and a second face forming a supportive surface, wherein a plurality of channels are formed between the first and second face by one or more joints. The channels are filled with a linked object in the form of a chain. The individual channel has a width such that one chain or link may pass another chain or link.

There is a need for a weight blanket that can be adjusted in a better way according to the user's body and thereby gives a better and more stimulating pressure on the user.

### Summary of the invention

Hence, one object of the present invention is to provide a blanket for therapeutic treatment that has better temperature regulation and handling ability.

A specific object of the present invention is to provide a blanket for therapeutic treatment that is easy to fold and wash.

One aspect of the present invention relates to a blanket for therapeutic treatment, the blanket (1) having a first face (21) and a second (22) face forming a supportive surface (2), wherein a plurality of channels (3) are formed between the first face (21) and the second (22) face by one or more joints (4), wherein at least one of the channels (3) being filled with objects (5) which adapt to the body of the user, and wherein the substantially spherical objects (5) exert a sporadic pressure on the body of the user through the supportive surface (1), characterized in that the objects (5) are substantially spherical objects (5) and in that the width of the individual channels (3) is of a size that does not allow one substantially spherical object (5A) to pass another substantially spherical object (5B) within said channels.

### Brief description of the figures

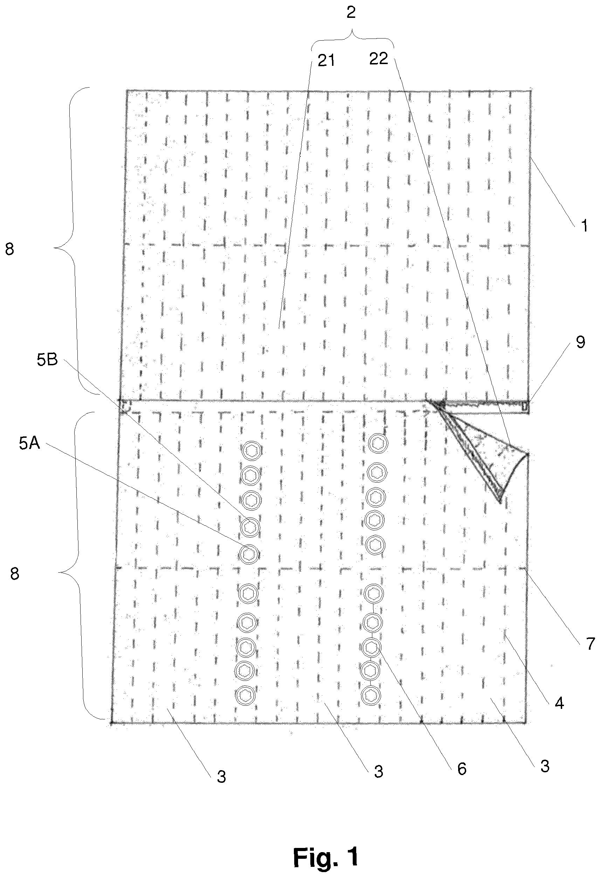

Figure 1 shows a preferred embodiment of a therapy blanket.

### Detailed description of the invention

One object of the present invention is to provide a blanket for therapeutic treatment that has better temperature regulation and handling ability.
The above object is solved by creating a blanket for therapeutic treatment with channels filled with substantially spherical objects. The reason for using substantially spherical objects is that the pressure upon the user's body should be sporadic to avoid obstruction of the blood circulation. Hence, the objects must be able to move easily in order not to apply pressure on the same point of the body. At the same time, the channels are constructed in such a way that the substantially spherical objects cannot lay on top of each other, nor can they clump together in only one region of the channel. In this way, it is easier to regulate the applied pressure on a specific region of the body. Furthermore, the blanket will not be as thick as it would be without this regulation. The reduced thickness results in a blanket that has a better temperature regulation.
One aspect of the present invention relates to a blanket for therapeutic treatment, the blanket (1) having a first face (21) and a second (22) face forming a supportive surface (2), wherein a plurality of channels (3) are formed between the first face (21) and the second (22) face by one or more joints (4), wherein at least one of the channels (3) being filled with objects (5) which adapt to the body of the user, and wherein the objects (5) exert a sporadic pressure on the body of the user through the supportive surface (1), characterized in that the objects (5) are substantially spherical objects (5) and in that the width of the individual channels (3) is of a size that does not allow one substantially spherical object (5A) to pass another substantially spherical object (5B) within said channels.
Another advantage of using substantially spherical objects is that they, due to the weight of the user, displace and form according to the shape of the body of the user, whereby a comfortable position can be obtained. The substantially spherical objects can be hollow and have a shell of polymer, glass, metal or the like, and have different kinds of filling such as fluids or solid materials.
In one embodiment, the substantially spherical objects are perfectly spherical, e.g. ball shaped.
The substantially spherical objects may be thermally isolating if the filling is warmed up by heat from the body of the user. The substantially spherical objects can also be solid and made from polymer, glass, metal or the like.
In another embodiment, the substantially spherical objects have a diameter in the range of 10 mm to 100 mm. The diameter of the substantially spherical objects can advantageously be varied in order to accommodate the different size of users, e.g. a child or an adult. The diameter of the substantially spherical objects can also be varied in order to exert a more or less sporadic pressure on the body of the user. To obtain the calming effect it is essential that the pressure exerted on the body of the user is sporadic.

To improve the production of the blanket, the substantially spherical objects can be connected linearly by e.g. a polymer string, which makes it easier to fill the channels.

Thus, in one embodiment, the substantially spherical objects (5) are connected linearly to form a string of substantially spherical objects (6).

To be able to wash the blanket, all components of the blanket should be suitable for washing in a washing machine. Hence, in one embodiment, the substantially spherical objects (5) are suitable for washing in a washing machine.

In another embodiment, the individual channels (3) run in the lengthwise and/or widthwise direction of the blanket.

In order to secure an evenly distribution of the substantially spherical objects along the individual channel, the blanket further comprises one or more structural barriers within the channels (3).

In another embodiment, the one or more structural barriers is/are a joint (7) between the first face (21) and the second (22) face of the supportive surface.

A specific object of the present invention is to provide a blanket for therapeutic treatment that is easy to fold and wash. This can be solved when the joint is a stitching, since such a barrier is of limited size. Hence, the combination of the channels with the stitching barrier prevent the substantially spherical objects from clumping together and obstructing the operation when a user want to roll-up or pack up the blanket, e.g. for transportation.

In one embodiment, one or more of the joints (7) is a stitching.

In another embodiment, one or more of the joints (7) is a zipper (9), thereby being able to divide the blanket into subunits (8) for easier handling. Washing is also made easier, since the subunits will more easily fit into the washing machine.

In yet another embodiment, the zipper (9) is positioned in the width wise direction of the blanket, thereby limiting its contact with the body of the user.

In still another embodiment, the channels (3) are evenly distributed over the whole surface of the blanket.

In one embodiment, the first and/or second face comprises one or more layers of fabric.

In still another embodiment, the first face (21) and/or the second (22) face comprise at least one layer of insulating material.

It should be noted that embodiments and features described in the context of one of the aspects of the present invention also apply to the other aspects of the invention.

The invention will now be described in further details in the following non-limiting examples.

### Examples

In Fig. 1, a preferred embodiment of a therapy blanket (1) according to the invention is shown. The blanket (1) has the same dimensions as an ordinary blanket, thus a length of about 200 cm and a width of about 140 cm. The blanket (1) includes the two faces (21) and (22) as two layers of fabric, between which channels (3) with a width of about 75 mm are made by sewing a number of joints (4) (stitching's/- seams). If necessary, these stitching's are doubled or tripled, in order to give firmness and smoothness to said channels (3).

The channels (3) are each filled with a number of hollow balls (5) made of a polymeric material, and have a diameter of 50 mm. This diameter makes sure that the hollow balls (5A and 5B) cannot lie on top of each other, nor can clump together in only one region of the channel. In this way, it is easier to regulate the applied pressure on a specific region of the body. Furthermore, the blanket will not be as thick as it would be without this regulation. The reduced thickness results in a blanket that has a better temperature regulation.

To improve the production of the blanket, the hollow balls can be connected linearly (6) by e.g. a polymer string, which makes it easier to fill the channels.

In order to secure an evenly distribution of the substantially spherical objects along the individual channel, the blanket further comprises three structural barriers within each channel (3). Here, two of the structural barriers are made by sewing a stitching (7). If necessary, these stitching's are doubled or tripled, in order to give firmness and smoothness to said channels (3). Such a barrier is of limited size, and the combination of the channels with the stitching barrier prevent the substantially spherical objects from clumping together and obstructing the operation when a user want to roll-up or pack up the blanket, e.g. for transportation.

The third structural barrier is made by inserting a joint in a form of a zipper (9), which allows the blanket to be divided into two subunits (8) for easier handling. Washing is thereby made easier, since the subunits will more easily fit into the washing machine.

The zipper (9) is positioned in the width wise direction of the blanket, thereby limiting its contact with the body of the user.

## Claims

1. A blanket for therapeutic treatment, the blanket (1) having a first face (21) and a second (22) face forming a supportive surface (2), wherein a plurality of channels (3) are formed between the first face (21) and the second (22) face by one or more joints (4), at least one of the channels (3) being filled with objects (5) which adapt to the body of the user, and wherein the objects (5) exert a sporadic pressure on the body of the user through the supportive surface (2), **characterized in that** the objects (5) are substantially spherical objects (5) and **in that** the width of the individual channels (3) is of a size that does not allow one substantially spherical object (5A) to pass another substantially spherical object (5B) within said channels.

2. A blanket according to claim 1, **characterized in that** the substantially spherical objects (5) are connected linearly to form a string of substantially spherical objects (6).

3. A blanket according to any one of the claims 1-2, **characterized in that** the substantially spherical objects (5) are suitable for washing in a washing machine.

4. A blanket according to any one of the claims 1-3, **characterized in that** the individual channels (3) run in the lengthwise and/or widthwise direction of the blanket.

5. A blanket according to any one of the claims 1-4, **characterized in that** the blanket further comprises one or more structural barriers within the channels (3) for securing an evenly distribution of the substantially spherical objects along the individual channel.

6. A blanket according to claim 5, **characterized in that** the one or more structural barriers is a joint (7) between the first face (21) and the second (22) face of the supportive surface.

7. A blanket according to claim 6, **characterized in that** the joint (7) is a stitching.

8. A blanket according to claim 6, **characterized in that in that** one or more of the joints (7) is a zipper (9), thereby being able to divide the blanket into subunits (8) for easier handling.

9. A blanket according to claim 8, **characterized in that** the zipper (9) is positioned in the widthwise direction of the blanket, thereby limiting its contact with the body of the user.

10. A blanket according to any one of the claims 1-9, **characterized in that** the channels (3) are evenly distributed over the whole surface of the blanket.

11. A blanket according to any of claims 1-10, **characterized in that** the first face (21) and/or the second (22) face comprise at least one layer of insulating material.

## Patentansprüche

1. Decke zur therapeutischen Behandlung, wobei die Decke (1) eine erste Fläche (21) und eine zweite (22) Fläche, bildend eine unterstützende Oberfläche (2), aufweist, wobei eine Mehrheit von Kanälen (3) zwischen der ersten Fläche (21) und der zweiten (22) Fläche durch eine oder mehrere Verbindungen (4) gebildet sind, wobei mindestens einer der Kanäle (3) mit Gegenständen (5) gefüllt ist, die sich an den Körper des Benutzers anpassen, und wobei die Gegenstände (5) einen sporadischen Druck auf den Körper des Benutzers durch die unterstützende Oberfläche (2) ausüben, **dadurch gekennzeichnet, dass** die Gegenstände (5) im Wesentlichen kugelförmige Gegenstände (5) sind, und dass die Breite der einzelnen Kanäle (3) eine Größe aufweist, die es nicht erlaubt, dass ein im Wesentlichen kugelförmiger Gegenstand (5A) an einem anderen im Wesentlichen kugelförmigen Gegenstand (5B) innerhalb der Kanäle vorbeikommen kann.

2. Decke nach Anspruch 1, **dadurch gekennzeichnet, dass** die im Wesentlichen kugelförmigen Gegenstände (5) linear verbunden sind, um eine Reihe von im Wesentlichen kugelförmigen Gegenständen (6) zu bilden.

3. Decke nach einem der Ansprüche 1-2, **dadurch gekennzeichnet, dass** die im Wesentlichen kugelförmigen Gegenstände (5) dafür geeignet sind, in einer Waschmaschine gewaschen zu werden.

4. Decke nach einem der Ansprüche 1-3, **dadurch gekennzeichnet, dass** sich die einzelnen Kanäle (3) in der Längsrichtung und/oder Breitenrichtung der Decke erstrecken.

5. Decke nach einem der Ansprüche 1-4, **dadurch gekennzeichnet, dass** die Decke weiter eine oder mehrere strukturelle Barrieren in den Kanälen (3) zum Sichern einer gleichmäßigen Verteilung von den im Wesentlichen kugelförmigen Gegenständen entlang dem einzelnen Kanal umfasst.

6. Decke nach Anspruch 5, **dadurch gekennzeichnet, dass** die eine oder mehrere strukturelle Barrieren eine Verbindung (7) zwischen der ersten Fläche (21) und der zweiten (22) Fläche der unterstützenden Oberfläche ist.

7. Decke nach Anspruch 6, **dadurch gekennzeichnet, dass** die Verbindung (7) eine Naht ist.

8. Decke nach Anspruch 6, **dadurch gekennzeichnet, dass** eine oder mehrere der Verbindungen (7) ein Reißverschluss (9) ist, wodurch die Decke in Untereinheiten (8) zur einfacheren Handhabung unterteilt werden kann.

9. Decke nach Anspruch 8, **dadurch gekennzeichnet, dass** der Reißverschluss (9) in der Breitenrichtung der Decke positioniert ist, wodurch sein Kontakt mit dem Körper des Benutzers begrenzt wird.

10. Decke nach einem der Ansprüche 1-9, **dadurch gekennzeichnet, dass** die Kanäle (3) über die ganze Oberfläche der Decke gleichmäßig verteilt sind.

11. Decke nach einem der Ansprüche 1-10, **dadurch gekennzeichnet, dass** die erste Fläche (21) und/oder die zweite (22) Fläche mindestens eine Schicht von Isoliermaterial umfassen.

## Revendications

1. Couverture pour un traitement thérapeutique, la couverture (1) ayant une première face (21) et une deuxième face (22) formant une surface de support (2), dans laquelle une pluralité de canaux (3) est formée entre la première face (21) et la deuxième face (22) par une ou plusieurs articulations (4), au moins l'un des canaux (3) étant remplis d'objets (5) s'adaptant au corps de l'utilisateur, et dans laquelle les objets (5) exercent une pression sporadique sur le corps de l'utilisateur à travers la surface de support (2), **caractérisée en ce que** les objets (5) sont des objets essentiellement sphériques (5), et **en ce que** la largeur des canaux individuels (3) est d'une taille qui ne permet pas à un objet essentiellement sphérique (5A) de passer un autre objet essentiellement sphérique (5B) dans lesdits canaux.

2. Couverture selon la revendication 1, **caractérisée en ce que** les objets essentiellement sphériques (5) sont reliés de manière linéaire pour former une chaîne d'objets essentiellement sphériques (6).

3. Couverture selon l'une quelconque des revendications 1 à 2, **caractérisée en ce que** les objets essentiellement sphériques (5) sont appropriés pour le lavage dans une machine à laver.

4. Couverture selon l'une quelconque des revendications 1 à 3, **caractérisée en ce que** les canaux individuels (3) s'étendent dans le sens de la longueur et/ou de la largeur de la couverture.

5. Couverture selon l'une quelconque des revendications 1 à 4, **caractérisée en ce que** la couverture comprend en outre un ou plusieurs obstacles structurels à l'intérieur des canaux (3) pour assurer une répartition régulière des objets essentiellement sphériques le long du canal individuel.

6. Couverture selon la revendication 5, **caractérisée en ce que** l'un ou plusieurs obstacles structurels est/sont une articulation (7) entre la première face (21) et la deuxième face (22) de la surface de support.

7. Couverture selon la revendication 6, **caractérisée en ce que** l'articulation (7) est une couture.

8. Couverture selon la revendication 6, **caractérisée en ce que** l'une ou plusieurs des articulations (7) est/sont une fermeture à glissière (9), étant ainsi en mesure de diviser la couverture en sous-unités (8) pour faciliter la manipulation.

9. Couverture selon la revendication 8, **caractérisée en ce que** la fermeture à glissière (9) est positionnée dans le sens de la largeur de la couverture, ce qui limite son contact avec le corps de l'utilisateur.

10. Couverture selon l'une quelconque des revendications 1 à 9, **caractérisée en ce que** les canaux (3) sont répartis de manière régulière sur toute la surface de la couverture.

11. Couverture selon l'une quelconque des revendications 1 à 10, **caractérisée en ce que** la première face (21) et/ou la deuxième face (22) comprend au moins une couche de matériau isolant.
